# DEMANDE DE BREVET EUROPEEN

(11) **EP 4 732 687 A1**
(43) Date de publication de la demande: **29.04.2026**
(21) Numéro de dépôt: 25207161.8
(22) Date de dépôt: 07.10.2025
(51) Int. Cl.: A24F 5/04, A61M 15/00, A61M 15/06

(54) **DISPOSITIF D'INHALATION**

(30) Priorité: 09.10.2024 FR 2410914
(71) Demandeur: Wancleme, 59000 Lille (FR)
(72) Inventeur: DESTOMBE, François, 59000 LILLE (FR)
(74) Mandataire: Plasseraud IP

(57) **Abrégé**

La présente divulgation est relative à un dispositif d'inhalation (1) de matières (M) solides ou pulvérulentes comprenant une enceinte sphéroïdale (2) plastique destinée à recevoir la matière à consommer; ladite enceinte sphérique communiquant fluidiquement avec un conduit d'inhalation (3) par une ouverture latérale (23) d'une paroi de ladite enceinte, ladite enceinte présentant un évent (21) formant une entrée d'air, ledit dispositif étant configuré pour sublimer la matière contenue dans ladite enceinte sphéroïdale (2) par une flamme (F) venant lécher la paroi d'un support résistant à la chaleur, agencé au niveau d'une ouverture inférieure de la ladite enceinte sphéroïdale plastique.

## Description

La présente divulgation est relative à un dispositif d'inhalation de matière solide ou pulvérulente comprenant une enceinte sphéroïdale destinée à recevoir la matière à consommer, ladite enceinte sphérique communiquant fluidiquement avec un conduit d'inhalation par une ouverture latérale de ladite enceinte, ladite enceinte présentant un évent formant une entrée d'air.

Un tel dispositif est configuré pour sublimer la matière contenue dans ladite enceinte par une flamme venant lécher ladite enceinte sphéroïdale et chauffer la matière par l'intermédiaire de la paroi de ladite enceinte.

### Domaine technique

Le domaine de la présente divulgation est celui des accessoires pour fumer, destinés à la consommation par inhalation de produits solides ou semi-solides (sous forme de poudres, pâtes, cailloux...).

La présente divulgation concerne plus particulièrement des accessoires développés dans le cadre de programmes de Réduction Des Risques (RDR) pour la santé, qui est une démarche de santé publique destinée à assurer une meilleure sécurité sanitaire des consommateurs de produits par inhalation, et notamment pour réduire le risque de transmission de maladies infectieuses par le sang, et limiter au maximum les risques de contaminations, voire de blessures, de ces consommateurs avec les accessoires nécessaires à la consommation de tels produits.

On connaît des accessoires employés pour la consommation par inhalation de tels produits et en particulier les pipes en verre de type borosilicate qui est une qualité de verre supérieur, plus épais, résistant aux températures élevées.

Une telle pipe en verre comprend un corps formant une enceinte destinée à recevoir en son intérieur un caillou/ou cristaux, ainsi qu'un évent formant une entrée d'air, et une partie tubulaire en communication avec le volume de l'enceinte, formant un embout d'inhalation.

Les cristaux ou cailloux dans l'enceinte sont consommés par sublimation, chauffés par une flamme d'un briquet au travers du verre de borosilicate qui est résistant à la flamme.

### Technique antérieure

L'état de la technique connait encore, du document FR3120499 un dispositif pour la consommation par inhalation de substances solides ou pulvérulentes sèches qui comporte un fourreau constitué d'un corps en verre de forme sphérique avec un fond aplati et s'ouvrant du côté diamétralement opposé audit fond par un col fileté d'une section comprise entre 15 et 30 millimètres, ledit corps en verre présentant un évent débouchant latéralement.

Ledit conduit d'inhalation débouchant à l'extrémité frontale d'un bouchon présentant un filetage complémentaire à celui dudit col, un élément filtrant constitué d'un ensemble de fils compressés en acier inoxydable engagé dans ledit conduit d'inhalation.

Selon les constatations de l'inventeur, un tel état de la technique en verre dans un seul tenant, ou tel qu'enseigné par FR3.120.499 comportant un corps en verre destiné à être chauffé à la flamme peut être amélioré, en particulier s'agissant vis-à-vis des risques de brûlures, et des risques de coupures.

### Résumé

La présente divulgation vient améliorer la situation.

Il est proposé un dispositif d'inhalation de matières solides ou pulvérulentes comprenant une enceinte sphéroïdale destinée à recevoir la matière à consommer; ladite enceinte sphérique communiquant fluidiquement avec un conduit d'inhalation par une ouverture latérale d'une paroi de ladite enceinte, ladite enceinte présentant au moins un évent formant une entrée d'air, ledit dispositif étant configuré pour sublimer la matière contenue dans ladite enceinte sphéroïdale par une flamme venant lécher la paroi de ladite enceinte sphéroïdale, caractérisé en ce que la paroi enceinte sphéroïdale est majoritairement en plastique et la paroi en plastique de ladite enceinte sphéroïdale présente une ouverture inférieure, pour un support résistant à la flamme configuré pour contenir les matières à consommer, ledit support résistant à la flamme étant dans un matériau distinct du plastique, le support résistant à la flamme venant prolonger la paroi plastique de l'enceinte sphéroïdale en obturant ladite ouverture inférieure. Les caractéristiques exposées dans les paragraphes suivants peuvent, optionnellement, être mises en œuvre, indépendamment les unes des autres ou en combinaison les unes avec les autres.

Selon un mode de réalisation, le matériau du support résistant à la flamme est un métal.

Selon un mode de réalisation, ladite ouverture inférieure est de diamètre compris entre 10 mm et 30 mm tel que 20 mm.

Selon un mode de réalisation, le support résistant à la flamme est une coupelle, comprenant :
- une concavité destinée à recevoir les matières, la concavité tournée vers l'intérieur de l'enceinte sphéroïdale,
- une convexité, opposée configurée pour venir en emboîtement dans ladite ouverture inférieure du plastique de l'enceinte,
- un épaulement périphérique configuré pour venir en appui contre une surface intérieure du plastique de l'enceinte sphéroïdale.

Selon un mode de réalisation, une paroi en plastique de l'enceinte sphéroïdale est équipée d'une garde comprenant une saillie extérieure ou plusieurs saillies extérieures, s'étendant vers le bas en périphérie de l'ouverture inférieure, ladite garde configurée pour empêcher ou limiter le risque de contact avec le support résistant à la flamme. En particulier, la garde comprend ladite saillie extérieure ou les saillies extérieures, s'étendant en périphérie de l'ouverture inférieure forme un piètement configuré pour venir en appui stable sur une surface de support horizontale lorsque ledit dispositif d'inhalation repose sur ladite surface horizontale.

Selon un mode de réalisation, ladite enceinte sphéroïdale comprend une demi-coque inférieure, plastique portant ladite ouverture inférieure, et une demi-coque supérieure, plastique portant ledit au moins un évent, la demi-coque inférieure et la demi-coque supérieure comprenant respectivement un premier bord d'assemblage et un deuxième bord d'assemblage configurés pour être assemblés par emboîtement élastique.

Selon un mode de réalisation, la demi-coque inférieure est dans un premier plastique, et la demi-coque supérieure est dans un deuxième plastique, le deuxième plastique présentant une température de fusion inférieure à la température de fusion du premier plastique.

Selon un mode de réalisation, la demi-coque supérieure et le conduit d'inhalation forment une pièce plastique d'un seul tenant, moulée par injection, l'extrémité libre du conduit d'inhalation étant de préférence obstruée par une carotte d'injection, destinée à être retirée à la première utilisation, ladite carotte formant un témoin de non-utilisation.

Selon un mode de réalisation, ladite enceinte plastique est transparente, au moins sur une partie supérieure de ladite enceinte, en particulier la demi-coque supérieure, étant dans un plastique transparent.

### Brève description des dessins.

D'autres caractéristiques, détails et avantages apparaîtront à la lecture de la description détaillée ci-après, et à l'analyse des dessins annexés, sur lesquels :
**Fig. 1**

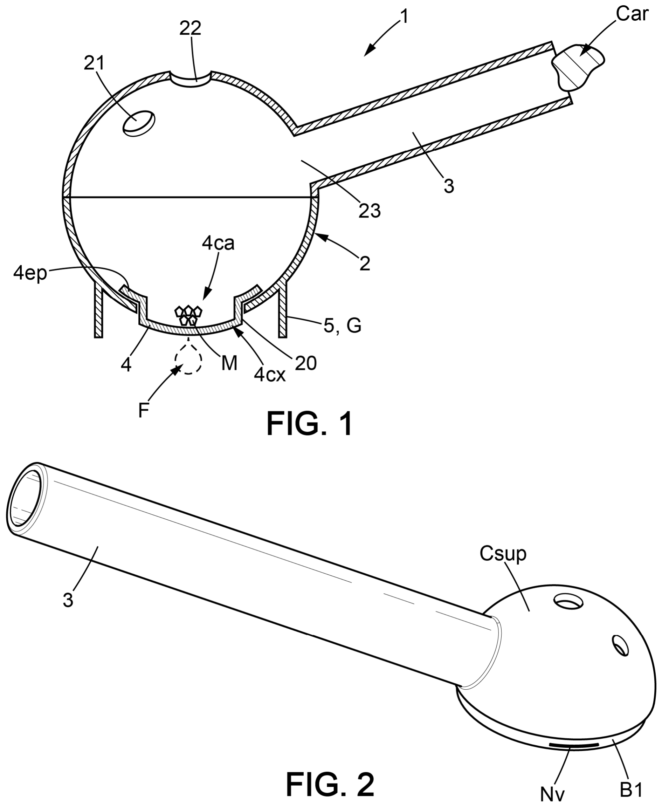

   [Fig. 1] est une vue schématique d'un dispositif d'inhalation de matières solides ou pulvérulentes, comprenant une enceinte sphéroïdale et une conduite d'inhalation en communication avec une ouverture latérale de ladite enceinte, le dispositif comprenant un assemblage de trois composants à savoir :
   - une première pièce plastique, typiquement moulée par injection formant une demi-coque supérieure et ladite conduite d'inhalation, la demi-coque supérieure comportant un évent d'entrée d'air, et une ouverture d'évacuation, supérieure, pour des fumées,
   - une deuxième pièce plastique, typiquement moulée par injection formant une demi-coque, inférieure, présentant une ouverture inférieure, ainsi qu'une garde formée par une saillie externe, entourant ladite ouverture inférieure,
   - un support résistant à la flamme, inséré dans la demi-coque inférieure, comprenant une coupelle, typiquement conformée à partir d'une feuille de métal, la coupelle logée en emboitement dans l'ouverture inférieure de la demi-coque inférieure, la coupelle comprenant un épaulement en appui sur la demi-coque inférieure autour de ladite ouverture, une concavité configurée pour recevoir la matière à consommer, ainsi qu'une convexité logée en emboitement dans ladite ouverture.
**Fig. 2**
   [Fig. 2] est une vue en perspective de détail de la première pièce plastique, typiquement d'un seul tenant, typiquement moulée par injection, formant non seulement la demi-coque supérieure, mais encore le conduit d'inhalation.
**Fig. 3**

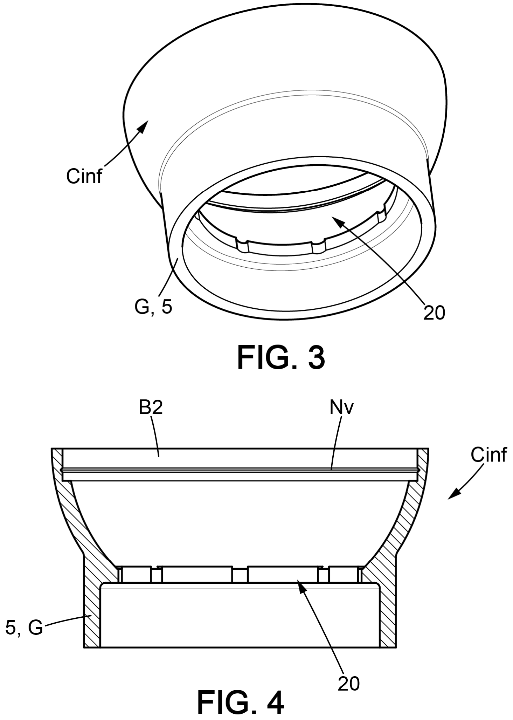

   [Fig. 3] est une vue en perspective de détail de la deuxième pièce plastique, typiquement d'un seul tenant.
**Fig. 4**
   [Fig. 4] est une vue de coupe de la demi-coque inférieure, passant d'un centre de l'ouverture inférieure.

### Description des modes de réalisation

Aussi, la présente divulgation est relative à un dispositif d'inhalation 1 de matières M solides ou pulvérulentes comprenant une enceinte sphéroïdale 2 destinée à recevoir la matière à consommer, ladite enceinte sphérique communiquant fluidiquement avec un conduit d'inhalation 3 par une ouverture latérale 23 d'une paroi de ladite enceinte sphéroïdale 2.

Ladite enceinte peut présenter un évent 21 ou 22 formant une entrée d'air, typiquement un évent formant un entrée d'air latérale.

Un tel dispositif est configuré pour sublimer la matière contenue dans ladite enceinte sphéroïdale 2 par une flamme F typiquement un briquet venant lécher la paroi de ladite enceinte sphéroïdale chauffant indirectement les matières contenues.

Selon la présente divulgation, la paroi de l'enceinte sphéroïdale 2 est majoritairement en plastique et la paroi en plastique de ladite enceinte sphéroïdale 2 présente une ouverture inférieure 20, pour un support 4 résistant à la flamme configuré pour contenir les matières M à consommer.

Ledit support résistant à la flamme S est dans un matériau distinct du plastique, le support 4 résistant à la flamme venant prolonger la paroi plastique de l'enceinte sphéroïdale en obturant ladite ouverture inférieure 20.

On obtient une enceinte sphéroïdale qui présente une paroi majoritairement en matière plastique, de préférence entièrement en matière plastique, à l'exception de l'ouverture supérieure 20 pour laquelle la paroi de l'enceinte sphéroïdale 2 est dans la matière résistante au feu dudit support 4 résistant au feu 4.

En utilisation, la flamme est configurée pour venir directement lécher principalement la matière résistant au feu du support, afin de sublimer la matière contenue dans le support.

Le matériau du support 4 peut être en verre, ou encore de préférence en métal, tel que par exemple l'aluminium. L'utilisation d'un support résistant à la flamme en métal dans le dispositif d'inhalation permet de garantir une meilleure résistance à la chaleur générée par la flamme utilisée pour sublimer la matière. Le métal, étant un excellent conducteur thermique, assure une distribution uniforme de la chaleur, ce qui améliore l'efficacité de la sublimation de la matière contenue dans le support.

En utilisation, l'utilisateur peut maintenir le dispositif d'inhalation par la partie de l'enceinte en plastique, d'une main, et de l'autre tenir un briquet.

Par comparaison à l'état de la technique des pipes entièrement en verre de type borosilicate, ou encore selon le document FR3.120.499 A1 pour lequel ladite enceinte contenant les matières est entièrement en verre, le dispositif d'inhalation présente pour avantage de pouvoir être maintenu par la partie plastique de l'enceinte qui est majoritaire, limitant non seulement les risques de brûlure par rapport au verre, mais encore les risques de coupure si le verre vient à être brisé.

En effet, l'enceinte en plastique reste froide au toucher, tandis que le support résistant à la flamme peut supporter une exposition directe à la flamme sans se dégrader.

La configuration où l'enceinte en plastique présente une ouverture inférieure pour le support résistant à la flamme permet un chauffage efficace de la matière par contact direct avec la flamme, assurant ainsi une sublimation efficace de la matière. Ce design simplifie également l'assemblage et le démontage du dispositif pour le nettoyage et l'entretien.

L'intégration d'un évent dans l'enceinte en plastique facilite le flux d'air à travers le dispositif, améliorant ainsi le processus d'inhalation et garantissant que la matière sublimée est efficacement aspirée par le conduit d'inhalation. Cela améliore l'expérience utilisateur globale en offrant une inhalation plus douce et mieux contrôlée.

De manière générale, ladite enceinte sphéroïdale 2 peut présenter une ouverture supérieure 22, qui est préférentiellement diamétralement opposée à l'ouverture inférieure 20.

De plus, cette disposition permet un chargement plus facile de la matière à consommer dans l'enceinte, car l'ouverture supérieure offre un accès direct et pratique. Cette ouverture peut former un évent, en particulier lorsque le dispositif est dépourvu dudit évent 21 formant l'entrée d'air latérale.

Le diamètre de l'ouverture supérieure 22 est de préférence supérieure au diamètre de l'évent 21 formant l'entrée d'air latéral, et de préférence inférieur au diamètre de l'ouverture 23 mettant en communication le volume de l'enceinte et la conduite d'inhalation 3.

L'ouverture supérieure 22 peut être une ouverture d'évacuation des fumées, ou encore être une ouverture autorisant le chargement de la matière dans ladite enceinte. La présence de l'ouverture supérieure 22, diamétralement opposée à l'ouverture inférieure 20 dans l'enceinte sphéroïdale permet une meilleure circulation de l'air et des fumées à l'intérieur du dispositif. Cette configuration facilite l'évacuation des fumées générées par la sublimation de la matière, améliorant ainsi l'efficacité du processus d'inhalation.

Selon un mode de réalisation, les dimensions du dispositif d'inhalation peuvent être les suivantes, en tout ou partie :
- Diamètre moyen de l'enceinte sphéroïdale 2 compris entre 18 mm et 70 mm, tel que 32 mm,
- Diamètre de l'ouverture latérale 23 et du conduit d'inhalation 3 compris entre 3 mm et 20 mm, tel que 8 mm,
- Diamètre de l'évent 21 compris entre 1,5 mm et 6 mm, tel que 3 mm,
- Diamètre de l'ouverture inférieure 20 compris entre 10 mm et 30 mm tel que 20 mm,
- Diamètre de l'ouverture supérieure 22 compris entre 3 mm et 10 mm tel que 5 mm.

Selon un mode de réalisation, le support 4 résistant à la flamme est une coupelle, peut comprendre :
- une concavité 4ca destinée à recevoir les matières M, la concavité tournée vers l'intérieur de l'enceinte sphéroïdale 2,
- une convexité 4cx opposée à la concavité, la convexité configurée pour venir en emboitement dans ladite ouverture inférieure 20 du plastique de l'enceinte,
- un épaulement 4ep périphérique configuré pour venir en appui contre une surface intérieure du plastique de l'enceinte sphéroïdale 2.

La concavité assure que les matières sont bien contenues et exposées de manière optimale à la chaleur, tandis que la convexité garantit un ajustement sécurisé et stable du support dans l'enceinte.

De plus, l'épaulement périphérique 4ep de la coupelle, configuré pour venir en appui contre une surface intérieure de l'enceinte sphéroïdale 2, renforce la stabilité et l'intégrité structurelle du dispositif. Cela empêche le support de se déplacer ou de se désaligner pendant l'utilisation, assurant ainsi une expérience d'inhalation plus sûre et plus fiable.

La coupelle peut être typiquement obtenue à partir d'une feuille de métal, par exemple d'aluminium, afin de former l'épaulement 4ep de la coupelle ainsi que la concavité 4ca et la convexité 4cx. La coupelle peut être typiquement obtenue par les techniques de découpe et d'emboutissage.

La concavité 4ca du support 4 résistant à la flamme permet de contenir les matières M à sublimer, alors que la convexité 4cx, logée dans l'ouverture inférieure 20, est configurée pour être directement en contact avec la flamme du briquet pour chauffer les matières M contenues dans la concavité 4ca.

Selon un mode de réalisation, une paroi en plastique de l'enceinte sphéroïdale 2 est équipée d'une garde G comprenant une saillie extérieure 5 ou une ou plusieurs saillies extérieures, s'étendant vers le bas en périphérie de l'ouverture inférieure 20, ladite garde configurée pour empêcher ou limiter le risque de contact avec le support S résistant à la flamme. La ou les saillies extérieures 5 de la garde 5 sont typiquement en plastique, d'un seul tenant avec la paroi plastique de l'enceinte sphéroïdale.

Comme illustrée à la figure 4, à titre d'exemple, la saillie externe 5 peut être sensiblement cylindrique, l'axe de révolution Ar du cylindre de la saillie sensiblement coaxial à un rayon de l'enceinte sphéroïdale 2 passant par un centre de l'ouverture inférieure 20.

Selon un mode de réalisation, la garde G comprenant ladite saillie extérieure 5 ou les saillies extérieures, peut former un piètement configuré pour venir en appui stable sur une surface de support horizontale lorsque ledit dispositif d'inhalation 1 repose sur ladite surface horizontale. Selon un mode de réalisation, une base du cylindre formant la garde G est configurée pour venir en appui sur la surface horizontale, dans une position stable du dispositif d'inhalation.

Ainsi l'ajout d'une garde G comprenant une ou plusieurs saillies extérieures 5 autour de l'ouverture inférieure 20 de l'enceinte sphéroïdale 2 offre une protection supplémentaire contre les risques de contact accidentel avec le support résistant à la flamme. Cette caractéristique réduit considérablement le risque de brûlures pour l'utilisateur, car elle crée une barrière physique entre la main de l'utilisateur et le support chauffé.

La garde G améliore ainsi la sécurité globale du dispositif en empêchant tout contact direct avec les parties les plus chaudes du dispositif pendant l'utilisation. Cela permet à l'utilisateur de manipuler le dispositif en toute confiance, sachant que les risques de blessures sont minimisés. Cette caractéristique contribue également à la stabilité du dispositif lorsqu'il est posé sur une surface, en offrant un appui supplémentaire. Cette configuration assure ainsi que le dispositif reste en position verticale et stable lorsqu'il est posé, réduisant ainsi le risque de renversement accidentel.

Selon un mode de réalisation, ladite enceinte sphéroïdale 2 peut comprendre une demi-coque inférieure Cinf, plastique portant ladite ouverture inférieure 20, et une demi-coque supérieure Csup, plastique portant l'évent 21, voire ladite ouverture supérieure.

La demi-coque inférieure Cinf et la demi-coque supérieure peuvent comprendre respectivement un premier bord d'assemblage B1, circulaire et un deuxième bord d'assemblage B2 circulaire, configurés pour être assemblés par emboitement élastique. Les premier et deuxième bords d'assemblage peuvent comprendre respectivement des nervures Nv, respectivement tournées vers l'extérieur, et tournées vers l'intérieur configurées pour s'accrocher mutuellement pour assurer un verrouillage de l'emboitement.

La conception de l'enceinte sphéroïdale 2 en deux demi-coques, une demi-coque inférieure Cinf portant l'ouverture inférieure 20 et une demi-coque supérieure Csup portant l'évent 21, permet un assemblage et un démontage faciles du dispositif. Les demi-coques peuvent être assemblées par emboîtement élastique grâce à des bords d'assemblage B1, B2 configurés pour s'accrocher mutuellement, assurant ainsi un verrouillage sécurisé.

Cette configuration modulaire facilite le nettoyage et l'entretien du dispositif, car les utilisateurs peuvent facilement séparer les deux demi-coques pour accéder à l'intérieur de l'enceinte. De plus, l'assemblage par emboîtement élastique permet de maintenir une étanchéité adéquate entre les deux parties, garantissant ainsi une performance optimale du dispositif pendant l'inhalation.

Selon un mode de réalisation, la demi-coque inférieure Cinf peut être dans un premier plastique, et la demi-coque supérieure Csup est dans un deuxième plastique. Le deuxième plastique peut présenter typiquement une température de fusion inférieure à la température de fusion du premier plastique. Le premier plastique de la demi-coque inférieure est de préférence dans un matériau susceptible de résister à des plus hautes températures que le deuxième plastique de la demi-coque supérieure.

Le fait que la demi-coque inférieure Cinf soit fabriquée dans un premier plastique, et la demi-coque supérieure Csup dans un deuxième plastique, où le deuxième plastique présente une température de fusion inférieure à celle du premier plastique, permet d'optimiser les propriétés thermiques et mécaniques de chaque partie du dispositif. Le premier plastique, plus résistant à la chaleur, assure que la demi-coque inférieure peut supporter des températures élevées sans se déformer, ce qui est avantageux pour la zone proche du support résistant à la flamme.

En revanche, l'utilisation d'un deuxième plastique avec une température de fusion inférieure pour la demi-coque supérieure permet de réduire les coûts de fabrication et d'améliorer la facilité de moulage par injection. Cette différenciation des matériaux permet d'obtenir un dispositif d'inhalation qui combine durabilité, résistance à la chaleur et efficacité de production, tout en maintenant une performance optimale pendant l'utilisation.

Par exemple, le premier plastique peut être un polyamide, en particulier un polyamide 6, et alors que le deuxième plastique peut être un PVC.

Le polyamide, tel que le polyamide 6, est un matériau qui offre une excellente résistance à la chaleur et aux températures élevées. Cette propriété est particulièrement bénéfique pour la demi-coque inférieure, qui est en contact direct avec le support résistant à la flamme. Le polyamide peut supporter des températures élevées sans se déformer, sans se dégrader rapidement, assurant ainsi la durabilité et la fiabilité du dispositif d'inhalation.

De plus, le polyamide possède une bonne résistance mécanique, ce qui renforce la structure de la demi-coque inférieure et améliore la stabilité globale du dispositif. Cette résistance mécanique permet également de mieux supporter les contraintes et les chocs éventuels lors de l'utilisation.

Le PVC (polychlorure de vinyle) est un matériau qui présente une température de fusion inférieure à celle du polyamide, ce qui facilite le processus de moulage par injection. L'utilisation du PVC pour la demi-coque supérieure permet de réduire les coûts de fabrication tout en maintenant une bonne qualité de production. Cela permet en particulier de mouler simultanément le conduit d'inhalation, et selon un mode de réalisation illustré.

Le PVC est également un matériau léger et facile à manipuler, ce qui améliore l'ergonomie du dispositif d'inhalation. De plus, le PVC offre une bonne résistance chimique, ce qui est avantageux pour la partie supérieure du dispositif qui peut être exposée à divers résidus de matière sublimée.

En combinant ces deux matériaux, le dispositif d'inhalation bénéficie des propriétés thermiques et mécaniques optimales du polyamide pour la demi-coque inférieure, tout en profitant des avantages économiques et de fabrication du PVC pour la demi-coque supérieure. Cette différenciation des matériaux permet d'obtenir un dispositif performant, durable et économique.

Selon un mode de réalisation, la demi-coque supérieure Csup et le conduit d'inhalation 3 peuvent former une pièce plastique d'un seul tenant, moulée par injection. La conception de la demi-coque supérieure Csup et du conduit d'inhalation 3 comme une pièce plastique d'un seul tenant, moulée par injection, simplifie le processus de fabrication et assure une intégrité structurelle accrue du dispositif. L'absence de joints ou de points de connexion entre ces deux éléments réduit les risques de fuites d'air ou de défaillances mécaniques, garantissant ainsi une performance fiable et constante pendant l'utilisation. L'extrémité libre du conduit d'inhalation peut être obstruée par une carotte d'injection Ci, destinée à être retirée à la première utilisation, ladite carotte formant un témoin de non-utilisation. Cela permet à l'utilisateur de vérifier que le dispositif est neuf et n'a pas été utilisé auparavant, assurant ainsi une hygiène et une sécurité accrues.

Selon un autre mode de réalisation, la dite enceinte sphéroïdale, en particulier la deuxième coque supérieure d'une part, et le conduit d'inhalation 3, d'autre part, peuvent comprendre deux pièces distinctes, les deux pièces étant assemblées l'une à l'autre, typiquement par emboitement au niveau de l'ouverture latérale 23,

Selon un mode de réalisation, ladite enceinte plastique 2 peut être transparente, au moins sur une partie supérieure de ladite enceinte, en particulier s'agissant la demi-coque supérieure Csup.

Le conduit d'inhalation 3 peut être dans un plastique transparent.

Un plastique transparent permet à l'utilisateur de visualiser directement la matière à consommer et de surveiller le processus de sublimation. Cette transparence améliore l'expérience utilisateur en offrant un contrôle visuel sur la quantité de matière restante et sur l'état de la matière pendant l'utilisation.

De plus, si le conduit d'inhalation 3 est également en plastique transparent, cela permet à l'utilisateur de voir le flux de fumée ou de vapeur, ce qui peut aider à ajuster l'intensité de l'inhalation et à optimiser l'utilisation du dispositif. La transparence des composants en plastique contribue également à une meilleure hygiène, car elle permet de détecter facilement les résidus ou les impuretés à l'intérieur du dispositif, facilitant ainsi le nettoyage et l'entretien.

### Liste des signes de référence

- 1. Dispositif d'inhalation,
- 2. Enceinte sphéroïdale,
- 20. Ouverture inférieure,
- 21. Event (notamment latéral) formant une entrée d'air,
- 22. Ouverture supérieure d'évacuation,
- 3. Conduit d'inhalation,
- 23. Ouverture latérale (de l'enceinte sphéroïdale)
- 4. Support résistant à la flamme
- 4ca. Concavité,
- 4cx. Convexité,
- 4ep. Epaulement,
- G. Garde,
- 5. Saillie extérieure (périphérique formant la garde, voire un piètement).

## Revendications

1. Dispositif d'inhalation (1) de matières (M) solides ou pulvérulentes comprenant une enceinte sphéroïdale (2) destinée à recevoir la matière à consommer; ladite enceinte sphérique communiquant fluidiquement avec un conduit d'inhalation (3) par une ouverture latérale (23) d'une paroi de ladite enceinte, ladite enceinte présentant au moins un évent (21, 22) formant une entrée d'air, ledit dispositif étant configuré pour sublimer la matière contenue dans ladite enceinte sphéroïdale (2) par une flamme (F) venant lécher la paroi de ladite enceinte sphéroïdale, **caractérisé en ce que** la paroi enceinte sphéroïdale (2) est majoritairement en plastique et la paroi en plastique de ladite enceinte sphéroïdale (2) présente une ouverture inférieure (20), pour un support (4) résistant à la flamme configuré pour contenir les matières (M) à consommer, ledit support résistant à la flamme (S) étant dans un matériau distinct du plastique, le support (4) résistant à la flamme venant prolonger la paroi plastique de l'enceinte sphéroïdale en obturant ladite ouverture inférieure (20).

2. Dispositif d'inhalation selon la revendication 1, dans lequel le matériau du support (4) résistant à la flamme est un métal.

3. Dispositif d'inhalation selon la revendication 1 ou 2, dans lequel ladite ouverture inférieure (20) est de diamètre compris entre 10 mm et 30 mm tel que 20mm.

4. Dispositif d'inhalation selon l'une des revendications 1 à 3 dans lequel le support résistant à la flamme est une coupelle, comprenant :
- une concavité (4ca) destinée à recevoir les matières (M), la concavité tournée vers l'intérieur de l'enceinte sphéroïdale (2),
- une convexité (4cx), opposée configurée pour venir en emboitement dans ladite ouverture inférieure (20) du plastique de l'enceinte,
- un épaulement (4ep) périphérique configuré pour venir en appui contre une surface intérieure du plastique de l'enceinte sphéroïdale (2).

5. Dispositif d'inhalation selon l'une des revendications 1 à 4, dans lequel une paroi en plastique de l'enceinte sphéroïdale (2) est équipée d'une garde (G) comprenant une saillie extérieure (5) ou plusieurs saillies extérieures, s'étendant vers le bas en périphérie de l'ouverture inférieure (20), ladite garde configurée pour empêcher ou limiter le risque de contact avec le support (S) résistant à la flamme.

6. Dispositif d'inhalation selon la revendication 5, dans lequel la garde (G) comprenant ladite saillie extérieure (5) ou les saillies extérieures, s'étendant en périphérie de l'ouverture inférieure forme un piètement configuré pour venir en appui stable sur une surface de support horizontale lorsque ledit dispositif d'inhalation (1) repose sur ladite surface horizontale.

7. Dispositif d'inhalation selon l'une des revendications 1 à 6, dans lequel ladite enceinte sphéroïdale (2) comprend une demi-coque inférieure (Cinf), plastique portant ladite ouverture inférieure (20), et une demi-coque supérieure (Csup), plastique portant ledit au moins un évent (21, 22), la demi-coque inférieure (Cinf) et la demi-coque supérieure comprenant respectivement un premier bord d'assemblage (B1) et un deuxième bord d'assemblage (B2) configurés pour être assemblés par emboitement élastique

8. Dispositif d'inhalation selon la revendication 7, dans lequel la demi-coque inférieure (Cinf) est dans un premier plastique, et la demi-coque supérieure (Csup) est dans un deuxième plastique, le deuxième plastique présentant une température de fusion inférieure à la température de fusion du premier plastique.

9. Dispositif d'inhalation selon la revendication 7 ou 8, dans lequel la demi-coque supérieure (Csup) et le conduit d'inhalation (3) forment une pièce plastique d'un seul tenant, moulée par injection, l'extrémité libre du conduit d'inhalation étant obstruée par une carotte d'injection (Ci), destinée à être retirée à la première utilisation, ladite carotte formant un témoin de non-utilisation.

10. Dispositif d'inhalation selon l'une des revendications 1 à 9, dans lequel ladite enceinte plastique (2) est transparente, au moins sur une partie supérieure de ladite enceinte plastique, en particulier la demi-coque supérieure (Csup) et éventuellement le conduit d'inhalation (3) lorsque le dispositif d'inhalation est selon l'une des revendications 7 à 9 est dans un plastique transparent.
